# EUROPÄISCHE PATENTSCHRIFT

(11) **EP 3 162 273 B1**
(45) Veröffentlichungstag und Bekanntmachung des Hinweises auf die Patenterteilung: **12.06.2019**
(21) Anmeldenummer: 16195440.9
(22) Anmeldetag: 25.10.2016
(51) Int. Cl.: A61B 1/00, A61B 1/055, G02B 23/24, H04N 5/225, H04N 13/296, H04N 13/239, A61B 1/04

(54) **OPTISCHES MEDIZINISCHES INSTRUMENT**
OPTICAL MEDICAL INSTRUMENT
INSTRUMENT MÉDICAL OPTIQUE

(30) Priorität: 26.10.2015 DE 102015118199
(43) Veröffentlichungstag der Anmeldung: 03.05.2017
(73) Patentinhaber: Karl Storz SE & Co. KG, 78532 Tuttlingen (DE)
(72) Erfinder: Czupalla, Christian, 78532 Tuttlingen (DE); Eisenkolb, Peter, 78532 Tuttlingen (DE); Heni, Andreas, 78532 Tuttlingen (DE); Kupferschmid, Markus, 78532 Tuttlingen (DE)

(56) Entgegenhaltungen:
- WO-A1-2011/113062
- WO-A1-2015/142797
- DE-A1-102013 215 422
- US-A1- 2013 250 061

## Beschreibung

Die vorliegende Erfindung ist auf ein optisches medizinisches Instrument oder ein anderes optisches Instrument bezogen, insbesondere auf ein Endoskop, ein Exoskop oder ein Operationsmikroskop, bei dem ein Bildsensor oder zwei Bildsensoren unabhängig voneinander zur Kompensation von Abbildungsfehlern und/oder zur Scharfstellung bei variierender Gegenstandsweite bewegbar oder verschiebbar sind.

Lange Zeit wies jedes medizinische Endoskop ein Okular auf, das einen unmittelbaren Blick durch das Endoskop ermöglichte. Später wurden Kameras entwickelt, die mit dem Okular eines Endoskops mechanisch verbunden und optisch gekoppelt werden konnten, um eine Wiedergabe des durch das Endoskop erfassten Bilds auf einem großen Bildschirm und optional seine Speicherung zu ermöglichen. Diese Modularität ermöglicht ferner ein Autoklavieren des Endoskops ohne Rücksicht auf die separate Kamera und eine Kombination der gleichen Kamera mit unterschiedlichen Endoskopen.

Ein flexibles Endoskop weist oft ein geordnetes Bündel von Lichtleitfasern auf und kann genauso wie ein starres Endoskop mit einer Kamera gekoppelt werden. Alternativ ist ein Bildsensor am distalen Ende des flexiblen Endoskops angeordnet. In US 2007/0100209 A1 ist ein Endoskop mit einem Bildsensor am distalen Ende beschrieben. Zur Bewegung einer bewegbaren Linse oder des Bildsensors in Richtung parallel zur optischen Achse der Linse ist ein Formgedächtnis-Element vorgesehen.

Stereoskopische Endoskope sind in US 5,588,948, EP 0 841 586 B1 und in WO 2014/012103 A1 beschrieben.

Aus der EP1514150B1 ist ein Endoskop zur gleichzeitigen Video- und Direkt-Beobachtung bekannt, mit einem Bildsensor und einem Strahlteiler. Bildsensor, Strahlteiler und Okularlinse sind auf einem beweglichen Bauteil angeordnet, das parallel zum Strahlengang der Direkt-Beobachtung durch Betätigung eines Brennpunktkranzes verschoben werden kann, um zu Fokussieren. Der Bildsensor ist derart seitlich des Strahlteilers an einer Lagersäule angebracht, dass das Instrument zusätzlichen Bauraum für den Sensor bereitstellen muss.

Aus der WO2011/113062A2 ist ein Stereoinstrument mit zwei Bildsensoren bekannt, die quer zu einer Längsrichtung des Instruments bewegt werden können.

Die Justage von Bildsensoren ist bei stereoskopischen Endoskopen mit zwei Bildsensoren auch angesichts sehr kleiner Querschnitte der Beobachtungsstrahlengänge besonders wichtig. Gleichzeitig sind aufgrund des geringen zur Verfügung stehenden Bauraums und der erwünschten Autoklavierbarkeit herkömmliche Konzepte nicht oder nur mit Einschränkungen auf stereoskopische Endoskope übertragbar.

Eine Aufgabe der vorliegenden Erfindung besteht darin, ein verbessertes optisches Instrument und ein verbessertes Verfahren zum Justieren eines Bildsensors eines optischen Instruments zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, bei einem stereoskopischen Endoskop oder einem anderen optischen Instrument einen Bildsensor an einem entlang eines vorbestimmten Pfads bewegbaren Schlitten zu befestigen, um eine Scharfstellung und/oder eine Korrektur eines optischen Fehlers einer optischen Einrichtung zu ermöglichen. Dabei wird insbesondere mittels einer reflektierenden Fläche das von der optischen Einrichtung ausgehende Licht um 90 Grad umgelenkt, um mit einer Bewegung des Bildsensors parallel zur optischen Achse der optischen Einrichtung eine Scharfstellung des Bilds zu ermöglichen.

Ein optisches Instrument umfasst eine optische Einrichtung zum Erzeugen eines reellen Bilds, einen Schlitten, der relativ zu der optischen Einrichtung entlang eines vorbestimmten Pfads bewegbar und arretierbar oder arretiert ist, und einen Bildsensor mit einer lichtempfindlichen Fläche zum Erfassen des reellen Bilds, wobei der Bildsensor an dem Schlitten befestigt ist.

Ein optisches Instrument umfasst zwei optische Einrichtungen zum Erzeugen zweier reeller Bilder, zwei Schlitten, die relativ zu den optischen Einrichtungen jeweils entlang eines vorbestimmten Pfads unabhängig voneinander bewegbar und optional arretierbar oder arretiert sind, und zwei Bildsensoren mit je einer lichtempfindlichen Fläche zum Erfassen von je einem der zwei reellen Bilder, wobei an jedem der zwei Schlitten einer der beiden Bildsensoren befestigt ist.

Ein optisches Instrument zur Erfassung von Stereobildern umfasst zwei optischen Einrichtungen zum Erzeugen je eines reellen Bilds, zwei am oder nahe dem proximalen Ende des optischen Instruments angeordnete Schlitten, die jeweils relativ zu den optischen Einrichtungen entlang eines vorbestimmten Pfads bewegbar und arretierbar sind, zwei Bildsensoren mit je einer lichtempfindlichen Fläche zum Erfassen des jeweiligen reellen Bilds, wobei an den Schlitten je einer der Bildsensoren befestigt ist, und jeweils eine reflektierende Fläche im Strahlengang zwischen der optischen Einrichtung und dem Bildsensor.

Ein optisches Instrument umfasst eine optische Einrichtung zum Erzeugen eines reellen Bilds, einen am oder nahe dem proximalen Ende des optischen Instruments angeordneten Schlitten, der relativ zu der optischen Einrichtung entlang eines vorbestimmten Pfads bewegbar und arretierbar oder arretiert ist, einen Bildsensor mit einer lichtempfindlichen Fläche zum Erfassen des reellen Bilds, wobei der Bildsensor an dem Schlitten befestigt ist, und eine reflektierende Fläche im Strahlengang zwischen der optischen Einrichtung und dem Bildsensor.

Das optische Instrument ist insbesondere ein optisches medizinisches Instrument. Das optische Instrument ist insbesondere ein Endoskop, ein Exoskop oder ein Operationsmikroskop. Ein Exoskop ist, ähnlich wie ein Operationsmikroskop, ein in der Medizintechnik verwendetes Instrument zum Beobachten eines menschlichen Körpers, dort insbesondere einer Operationsstelle oder des Körperinneren, von außerhalb des Körpers. Derartige Exoskope sind beispielsweise aus der EP2162050B1 bekannt. Das optische Instrument ist insbesondere ein stereoskopisches optisches Instrument zum Erfassen zweier Bilder durch zwei nebeneinander liegende Eintrittspupillen, die bei geeigneter Wiedergabe eine räumliche Wahrnehmung ermöglichen.

Die optische Einrichtung umfasst insbesondere ein Objektiv oder eine oder mehrere Linsen zum Erzeugen des reellen Bilds. Die optische Einrichtung kann hinter einem Relaislinsensystem (beispielsweise Stablinsensystem nach H. H. Hopkins) angeordnet sein oder ein solches Relaislinsensystem umfassen. Angaben zur Anordnung einer optischen Einrichtung vor oder hinter einer anderen optischen Einrichtung sind hier immer auf die Ausbreitungsrichtung von Licht, das von einem zu beobachtenden Gegenstand ausgeht, bezogen. Nach einem Relaislinsensystem angeordnet ist die optische Einrichtung insbesondere zur vergrößernden Erzeugung eines reellen Bilds vorgesehen und ausgebildet. Das mittels des Bildsensors zu erfassende Bild ist also insbesondere größer als das letzte vom Relaislinsensystem erzeugte reelle Bild (das bezogen auf das gesamte optische Instrument ein reelles Zwischenbild ist). Ein optisches Instrument zur Erfassung von Stereobildern umfasst insbesondere zwei Relaislinsensysteme, oder auch zwei Objektive.

Wenn das optische Instrument - beispielsweise ein Endoskop - einen Schaft und eine Handhabungseinrichtung am proximalen Ende des Schafts umfasst, ist der Schlitten insbesondere dann am proximalen Ende des optischen Instruments angeordnet, wenn er in der Handhabungseinrichtung angeordnet ist. In anderen Fällen ist der Schlitten insbesondere dann am oder nahe dem proximalen Ende des optischen Instruments angeordnet, wenn der Abstand des Massenschwerpunkts des Schlittens vom proximalen Ende des optischen Instruments nicht größer ist als sein Abstand (oder die Hälfte seines Abstands oder ein Drittel seines Abstands oder ein Fünftel seines Abstands oder ein Siebtel seines Abstands) vom distalen Ende des optischen Instruments.

Der Schlitten kann arretierbar oder arretiert sein. Wenn der Schlitten arretiert ist, ist mit der Bewegbarkeit des Schlittens die Bewegbarkeit des Schlittens bei Wegfall der Arretierung gemeint.

Der Schlitten ist insbesondere so geführt, dass er lediglich in einer Richtung, d.h. nur entlang eines einzigen geradlinigen Pfads vorbestimmter Länge, bewegbar oder verschiebbar ist. Der Schlitten ist insbesondere so spiel- und reibungsarm geführt, dass er nicht oder nicht wesentlich schwenkbar oder kippbar ist. Der Schlitten ist dann nicht wesentlich schwenk- oder kippbar, wenn er nur innerhalb eines Winkelbereichs schwenk- oder kippbar ist, innerhalb dessen eine solche Bewegung keinen wesentlichen Einfluss auf die Schärfe des mittels des Bildsensors erfassten Bilds hat. Dies ist insbesondere dann der Fall, wenn jeder Punkt der lichtempfindlichen Fläche des Bildsensors in einer Richtung orthogonal zur lichtempfindlichen Fläche maximal auf einer Strecke bewegt wird, die kleiner oder wesentlich kleiner als die Schärfentiefe ist.

Der Schlitten ist nur in Richtung parallel zur Längsachse des optischen Instruments bewegbar. Wenn das optische Instrument ein Endoskop mit starrem und geradem Schaft ist, ist der Schlitten insbesondere parallel zur Längsachse des Schafts bewegbar.

Bei einem optischen Instrument zur Erfassung von Stereobildern sind insbesondere zwei Schlitten unabhängig voneinander wie zuvor beschrieben auch gegeneinander bewegbar und arretierbar oder arretiert. Die Schlitten können insbesondere in unterschiedlichen Positionen arretiert werden.

Der Schlitten kann durch eine Leiterplatte bzw. Platine (engl.: PCB = Printed Circuit Board) aus faserverstärktem Kunststoff oder einem anderen Material, an oder in der Leitungen oder elektrisch leitfähige Pfade vorgesehen sind, gebildet sein. Alternativ kann der Schlitten aus Metall, Keramik, Kunststoff oder einem anderen elektrisch leitfähigen oder elektrisch isolierenden Material gebildet sein, ohne gleichzeitig Leitungen oder andere elektrisch leitfähige Pfade zu umfassen oder Teil eines Stromkreises zu sein. Eine Ausgestaltung des Schlittens ohne die Funktion einer Leiterplatte - beispielsweise aus Metall, Keramik oder einem geeigneten Kunststoff- kann eine besonders präzise Fertigung des Schlittens und eine besonders spiel- und reibungsarme Führung des Schlittens ermöglichen.

Der Bildsensor ist insbesondere zum direkten Erfassen des von der optischen Einrichtung erzeugten reellen Bilds vorgesehen, ausgebildet und angeordnet. Zwischen der optischen Einrichtung und dem Bildsensor ist also keine weitere abbildende Einrichtung vorgesehen. Der Bildsensor ist zum Erzeugen eines - insbesondere elektrischen - Signals, das das reelle Bild repräsentiert, vorgesehen und ausgebildet.

Der Bildsensor kann unmittelbar oder mittelbar (beispielsweise durch eine Leiterplatte) an dem Schlitten befestigt sein. Der Bildsensor ist insbesondere durch eine Klebung, eine Lötverbindung und/oder auf andere stoff-, form- und/oder kraft- bzw. reibschlüssige Weise an dem Schlitten befestigt. Wenn das optische Instrument ein Endoskop ist, ist der Bildsensor mit Schlitten insbesondere in einer Handhabungseinrichtung am proximalen Ende des Endoskops angeordnet.

Die reflektierende Fläche reflektiert insbesondere innerhalb des durch den Bildsensor zu erfassenden Wellenlängenbereichs unabhängig oder im Wesentlichen unabhängig von der Wellenlänge. Die reflektierende Fläche ist also insbesondere zum Umlenken des (sowohl hinsichtlich des Querschnitts als auch der Wellenlänge) gesamten Lichts vorgesehen, ausgebildet und angeordnet. Die reflektierende Fläche ist insbesondere zum Umlenken des Strahlengangs um 90 Grad vorgesehen und angeordnet. Die reflektierende Fläche ist insbesondere eben, weist also keine Brechkraft auf und verändert die Gegenstandsweite nicht. Die reflektierende Fläche ist insbesondere in oder an einem Umlenkprisma vorgesehen.

Die Anordnung des Bildsensors an einem Schlitten kann eine einfache Justierung des Bildsensors und eine einfache Kompensation von Abbildungsfehlern (insbesondere Brennweitefehlern) der optischen Einrichtung und/oder eines Relaislinsensystems oder anderer optischer Einrichtungen, die von Licht durchlaufen werden, das danach auf den Bildsensor fällt, ermöglichen. Alternativ oder zusätzlich kann die Anordnung des Bildsensors an einem Schlitten eine einfache Fokussierung bei variierender Gegenstandsweite ermöglichen. Eine Umlenkung des Strahlengangs durch eine reflektierende Fläche - insbesondere um 90 Grad - kann zu einer besonders kompakten und robusten Bauweise beitragen.

Bei einem optischen Instrument zur Erfassung von Stereobildern können durch die Anordnung der Bildsensoren an den Schlitten die Sensoren insbesondere einfach und unabhängig voneinander justiert werden. In den zwei Stereokanälen, beispielsweise in zwei Objektiven oder zwei Relaislinsensystemen, durch Toleranzen erzeugte optische Abbildungsfehler können somit unabhängig voneinander korrigiert werden. Auf diese Weise wird es möglich ein nahezu fehlerfreies stereoskopisches Bild zu erzeugen.

Bei einem optischen Instrument, wie es hier beschrieben ist, ist die reflektierende Fläche insbesondere mit dem Bildsensor starr verbunden und zusammen mit dem Bildsensor bewegbar.

Die reflektierende Fläche ist insbesondere an oder in einem Prisma vorgesehen, das auf die lichtempfindliche Oberfläche des Bildsensor geklebt oder auf andere Weise mit dem Bildsensor mechanisch starr verbunden ist. Der Bildsensor und die reflektierende Fläche bilden auf diese Weise eine starre mechanische Einheit, die mechanisch robust ist und eine einfache Handhabung ermöglicht. Gleichzeitig ist die mechanisch empfindliche lichtempfindliche Oberfläche des Bildsensors geschützt.

Bei einem optischen Instrument, wie es hier beschrieben ist, ist der Schlitten insbesondere in einer Richtung parallel zu der lichtempfindlichen Fläche des Bildsensors bewegbar.

Eine Bewegbarkeit oder Verschiebbarkeit des Schlittens in einer Richtung (insbesondere ausschließlich in einer Richtung) parallel zu der lichtempfindlichen Fläche des Bildsensors ist beispielsweise durch eine im Wesentlichen plattenförmige Ausgestaltung des Schlittens erzielbar, die einen geringen Bauraum in Anspruch nimmt und gleichzeitig mechanisch besonders steif und robust sein kann.

Bei einem optischen Instrument, wie es hier beschrieben ist, ist die lichtempfindliche Fläche des Bildsensors insbesondere parallel zur optischen Achse der optischen Einrichtung angeordnet.

Mit der optischen Einrichtung ist insbesondere die optische Achse der optischen Einrichtung an ihrer Lichtaustrittsfläche gemeint. Eine Umlenkung des von der optischen Einrichtung ausgehenden Lichts mittels einer reflektierenden Fläche oder auf andere Weise um 90 Grad kann die Anordnung der lichtempfindlichen Fläche des Bildsensors parallel zur optischen Achse der optischen Einrichtung ermöglichen.

Bei einem optischen Instrument, wie es hier beschrieben ist, ist die optische Achse des Strahlengangs zwischen der optischen Einrichtung und der reflektierenden Fläche insbesondere parallel zu der lichtempfindlichen Fläche des Bildsensors.

Ein optisches Instrument, wie es hier beschrieben ist, umfasst insbesondere ferner eine Klemmeinrichtung zum kraftschlüssigen Arretieren des Schlittens.

Die Klemmeinrichtung zum kraft- bzw. reibschlüssigen Arretieren des Schlittens ist insbesondere zur lösbaren Arretierung des Schlittens bei einer innerhalb eines vorbestimmten Bereichs beliebigen Position vorgesehen und ausgebildet. Die Klemmeinrichtung ist insbesondere so ausgebildet, dass die von der Klemmeinrichtung bei der vorgesehenen Verwendung erzeugte Normalkraft eine Haftreibungskraft erzeugt, die größer ist als die bei der vorgesehenen Verwendung des optischen Instruments auftretenden Kräfte auf den Schlitten in Richtung parallel zu der Richtung, in der er bewegbar ist.

Die Klemmeinrichtung umfasst insbesondere eine Madenschraube oder eine andere Schraube, die in ein Gewinde mit in einer Richtung abnehmendem Durchmesser oder in ein Gewinde in einer Bohrung mit in einer Richtung abnehmendem Durchmesser im Schlitten eingreift. Der Schlitten weist im Bereich des Gewindes oder der Bohrung mit dem Gewinde insbesondere einen Schlitz auf, der eine Verformung des Schlittens durch eine Aufweitung des Gewindes durch die Schraube ermöglicht.

Die Klemmeinrichtung ist insbesondere nahe einem geraden Randabschnitt oder in einer wulstförmigen Verdickung an einem geraden Randabschnitt, der parallel zur vorbestimmten Bewegungsrichtung des Schlittens ist, angeordnet.

Bei einem optischen Instrument zur Erfassung von Stereobildern sind insbesondere zwei Klemmeinrichtungen vorgesehen, die eine zuvor beschriebene Arretierung der Schlitten unabhängig voneinander ermöglichen.

Ein optisches Instrument, wie es hier beschrieben ist, umfasst insbesondere ferner eine Feder oder eine andere elastische Einrichtung zum Schieben des Schlittens in eine vorbestimmte Richtung.

Die vorbestimmte Richtung ist parallel zur optischen Achse der optischen Einrichtung zum Erzeugen eines reellen Bilds und/oder zur Längsrichtung des optischen Instruments. Die Feder ist insbesondere nahe einem geraden Randabschnitt oder in einer wulstförmigen Verdickung an einem geraden Randabschnitt, der parallel zur vorbestimmten Bewegungsrichtung des Schlittens ist, angeordnet. Die Feder ist insbesondere eine Druckfeder, die den Schlitten nach proximal schiebt, wobei der Schlitten manuell und/oder durch ein Werkzeug oder mittels eines Werkzeugs gegen die elastische Kraft der Feder nach distal bewegbar ist, solange er nicht mittels der beschriebenen Klemmeinrichtung oder auf andere Weise arretiert ist.

Mit einer derartigen Feder muss zur Justage des optischen Instruments auf den Schlitten nur in einer Richtung Kraft ausgeübt werden, um ihn - durch Variation der Kraft - in zwei entgegengesetzten Richtungen zu bewegen.

Ein optisches Instrument, wie es hier beschrieben ist, umfasst insbesondere ferner eine Stellschraube zum Bewegen des Schlittens.

Die Stellschraube ist insbesondere nahe einem geraden Randabschnitt oder in einer wulstförmigen Verdickung an einem geraden Randabschnitt, der parallel zur vorbestimmten Bewegungsrichtung des Schlittens ist, angeordnet. Das distale Ende der Stellschraube liegt insbesondere an einer distal des Schlittens angeordneten Wand an.

Die Stellschraube kann ein präzises Einstellen der Position des Schlittens ermöglichen. Durch Rotation der Stellschraube im Uhrzeigersinn kann der Schlitten fein dosiert nach proximal, durch Rotation der Stellschraube gegen den Uhrzeigersinn und gleichzeitiges Erzeugen einer den Schlitten nach distal schiebenden Kraft kann der Schlitten fein dosiert nach distal bewegt oder verschoben werden.

Bei einem optischen Instrument zur Erfassung von Stereobildern sind insbesondere zwei Stellschrauben vorgesehen, mit denen die zwei Schlitten unabhängig voneinander und auch gegeneinander verstellt werden können.

Ein optisches Instrument, wie es hier beschrieben ist, umfasst insbesondere ferner eine Antriebseinrichtung zum Bewegen des Schlittens.

Die Antriebseinrichtung umfasst insbesondere einen Linearmotor, einen Ultraschallmotor oder einen motorischen Spindelantrieb, der ein Bewegen des Schlittens gesteuert durch ein - insbesondere elektrisches - Steuersignal ermöglicht. Eine derartige Antriebseinrichtung ermöglicht eine Fokussierung bzw. ein Scharfstellen des mittels des Bildsensors erfassten Bilds bei variierender Gegenstandsweite.

Ein optisches Instrument zur Erfassung von Stereobildern umfasst insbesondere zwei Antriebseinrichtungen, die es ermöglichen, die Schlitten unabhängig voneinander und auch gegeneinander zu bewegen.

Ein optisches Instrument, wie es hier beschrieben ist, umfasst insbesondere ferner eine Leiterplatte, die mit dem Schlitten verbunden ist, wobei der Bildsensor an der Leiterplatte befestigt ist.

Ein optisches Instrument, wie es hier beschrieben ist, umfasst insbesondere ferner eine Leiterplatte, die mit dem Schlitten verbunden ist, wobei der Bildsensor an der Leiterplatte befestigt ist, und wobei die Leiterplatte einen flexiblen gekrümmten Abschnitt aufweist, der einen Längenausgleich bei Bewegung des Schlittens ermöglicht.

Die Leiterplatte bzw. Platine kann eine oder mehrere Lagen von elektrisch leitfähigen Leiterbahnen an oder in einem elektrisch isolierenden Material aufweisen. Die Leiterplatte kann teilweise starr oder vollständig flexibel sein, wobei insbesondere derjenige Teil der Leiterplatte, an dem der Bildsensor angeordnet ist, durch eine flächige Klebeverbindung mit dem Schlitten oder auf andere Weise relativ zum Schlitten unbewegt und unverformt gehalten wird.

Der flexible gekrümmte Abschnitt kann derart gekrümmt sein, dass er in seinem mechanisch spannungsärmsten Zustand (insbesondere vor Einbau in das optische Instrument) bereits gekrümmt ist, beispielsweise eine doppel-S-förmige Schleife bildet. Alternativ kann der flexible gekrümmte Abschnitt in seinem mechanisch spannungsärmsten Zustand eine gerade oder ebene oder im Wesentlichen gerade oder ebene Gestalt aufweisen und erst nach Einbau in das optische Instrument eine gekrümmte Gestalt annehmen.

Bei Ausgestaltung der Leiterplatte mit einem flexiblen gekrümmten Abschnitt, der einen Längenausgleich ermöglicht, sind keine flexiblen Kabelverbindungen zwischen der Leiterplatte und weiteren elektrischen oder optischen Einrichtungen, die innerhalb des optischen Instruments starr bzw. unbeweglich angeordnet sind, erforderlich. Vielmehr kann beispielsweise an einem vom Bildsensor abgewandten Ende des flexiblen gekrümmten Abschnitts der Leiterplatte ein Steckverbinder vorgesehen sein, der unmittelbar mit einem korrespondierenden Steckverbinder an einem weiteren elektrischen oder elektronischen Bauteil oder einer weiteren Leiterplatte innerhalb des optischen Instruments verbunden ist.

Bei einem optischen Instrument zur Erfassung von Stereobildern sind insbesondere zwei Leiterplatten wie oben beschrieben, die jeweils mit einem der Bildsensoren verbunden sein können.

Ein optisches Instrument, wie es hier beschrieben ist, ist insbesondere ein Endoskop mit einem starren Schaft. Endoskope mit elektronischen Bildsensoren werden auch als Videoendoskope bezeichnet.

Das Endoskop ist insbesondere ein medizinisches Endoskop, ist also zur Anwendung bei medizinischen Maßnahmen vorgesehen und ausgebildet. Alternativ kann das Endoskop für technische Anwendungen vorgesehen und ausgebildet sein. Der Schaft ist insbesondere gerade.

Bei einem optischen Instrument, wie es hier beschrieben ist, umfasst die optische Einrichtung insbesondere ein Stablinsensystem oder ein anderes Relaislinsensystem.

Ein optisches Instrument, wie es hier beschrieben ist, umfasst insbesondere ferner ein Führungsbauteil, das mit der optischen Einrichtung starr verbunden ist, wobei das Führungsbauteil zwei Nuten aufweist, in denen zwei voneinander abgewandte und parallele gerade Randabschnitte des Schlittens geführt sind.

Das Führungsbauteil kann mittelbar oder unmittelbar mit der optischen Einrichtung starr verbunden sein. Das Führungsbauteil weist insbesondere die Funktion einer Gleithülse auf, in der der Schlitten spiel- und reibungsarm geführt ist. Die Nuten im Führungsbauteil sind insbesondere zwei einander zugewandte parallele Nuten in der Wand eines zylindrischen Kanals im Führungsbauteil. Der zylindrische Kanal weist von den Nuten abgesehen beispielsweise im Wesentlichen einen kreisförmigen oder rechteckigen Querschnitt auf. Während der Kanal beispielsweise gebohrt oder gefräst sein kann, sind die Nuten insbesondere durch Elektroerosion erzeugt, um die erwünschte und für eine spiel- und reibungsarme Führung des Schlittens erforderliche Geometrie mit hoher Präzision zu gewährleisten.

Ein derartiges Führungsbauteil kann gleichzeitig eine kompakte und mechanisch steife und robuste Ausgestaltung des optischen Instruments ermöglichen.

Ein optisches Instrument, wie es hier beschrieben ist, ist insbesondere zur Erfassung von Stereobildern ausgebildet und umfasst zwei optische Einrichtungen zum Erzeugen je eines reellen Bilds, zwei Bildsensoren zum Erfassen je eines reellen Bilds und zwei Schlitten, an denen je ein Bildsensor angeordnet ist.

Die optischen Einrichtungen umfassen insbesondere jeweils ein Stablinsensystem oder ein anderes Relaislinsensystem, wobei die Relaislinsensysteme parallel in einem Schaft des optischen Instruments angeordnet sind.

Viele hier beschriebene Eigenschaften und Merkmale optischer Instrumente sind gerade im Fall eines optischen Instruments zur Erfassung von Stereobildern besonders vorteilhaft, weil sie eine Miniaturisierung und damit eine Einsparung von Bauraum ermöglichen. Ferner ist gerade bei zwei optischen Einrichtungen zum Erzeugen je eines reellen Bilds eine Justage der Bildsensoren unabhängig voneinander mittels je eines Schlittens besonders wichtig, um unterschiedliche optische Eigenschaften der beiden optischen Einrichtungen sowie ggf. vor den optischen Einrichtungen angeordneter Relaislinsensysteme oder anderer optischer Einrichtungen korrigieren zu können.

Bei einem optischen Instrument, wie es hier beschrieben ist, ist insbesondere in jeder der zwei Nuten je ein Randabschnitt von jedem der zwei Schlitten nebeneinander angeordnet.

Jede der beiden Nuten nimmt also zwei Randabschnitte und zwar von jedem der beiden Schlitten jeweils einen Randabschnitt auf. Der Aufwand für die spiel- und reibungsarme Führung beider Schlitten muss damit gegenüber einer Situation mit lediglich einem Schlitten nicht verdoppelt werden.

Bei einem optischen Instrument, wie es hier beschrieben ist, liegen die zwei Schlitten insbesondere flächig aneinander an.

Alternativ können die zwei Schlitten beispielsweise nur in den Bereichen ihrer parallelen geraden Randabschnitte (die insbesondere jeweils paarweise gemeinsam in den Nuten geführt sind) aneinander anliegen.

Bei einem optischen Instrument, wie es hier beschrieben ist, sind die zwei Bildsensoren insbesondere an voneinander abgewandten Seiten der zwei Schlitten angeordnet.

Die zwei Schlitten mit den zwei Bildsensoren und ggf. zwei Leiterplatten zwischen den Schlitten und den Bildsensoren sind insbesondere spiegelsymmetrisch zu einer zwischen den zwei Schlitten angeordneten Symmetrieebene oder rotationssymmetrisch bezüglich einer zwischen den Schlitten angeordneten zweizähligen Symmetrieachse. Baugruppen aus je einem Schlitten, ggf. einer Leiterplatte, einem Bildsensor und ggf. einem Prisma oder einem anderen optischen Bauteil mit einer reflektierenden Fläche können somit identisch ausgebildet sein und müssen nicht nach linkem oder rechtem Kanal unterschieden werden.

Die Bauteile werden durch die zuvor genannten Maßnahmen besonders kompakt und platzsparend angeordnet, so dass sie beispielsweise im Handgriff eines Videoendoskops untergebracht werden können, ohne dass dieser größer dimensioniert werden muss, als ergonomisch sinnvoll ist.

Bei einem Verfahren zum Justieren eines Bildsensors eines optischen Instruments wird ein Bild eines an einer vorbestimmten Position angeordneten Gegenstands erzeugt und der Bildsensor bis zu einer Position, bei der der Bildsensor das Bild scharf erfasst, bewegt, wobei bei dem Schritt des Bewegens der Bildsensor parallel zu einer lichtempfindlichen Oberfläche des Bildsensors bewegt wird.

Bei einem Verfahren zum Justieren eines Bildsensors eines optischen Instruments werden ein Bild eines an einer vorbestimmten Position angeordneten Gegenstands erzeugt, der Bildsensor bis zu einer Position, bei der der Bildsensor das Bild scharf erfasst, bewegt und ein Schlitten mit dem Bildsensor bei der Position, bei der der Bildsensor das Bild scharf erfasst, arretiert, wobei bei dem Schritt des Bewegens der Bildsensor parallel zu einer lichtempfindlichen Oberfläche des Bildsensors bewegt wird.

Vor oder bei dem Schritt des Bewegens des Bildsensors kann bereits ermittelt werden, ob der Bildsensor das Bild scharf erfasst. Schritte des Ermittelns, ob der Bildsensor das Bild scharf erfasst, und des Bewegens des Bildsensors können alternierend wiederholt oder gleichzeitig ausgeführt werden bis der Bildsensor das Bild scharf erfasst.

Der Schlitten mit dem Bildsensor wird insbesondere durch Klemmen arretiert.

Die beschriebenen Verfahren zum Justieren eines Bildsensors eines optischen Instruments können jeweils Teil eines Verfahrens zum Herstellen eines optischen Instruments sein und insbesondere auf optische Instrumente, wie sie hier beschrieben sind, bzw. bei diesen optischen Instrumenten angewendet werden.

### Kurzbeschreibung der Figuren

Nachfolgend werden Ausführungsformen anhand der beigefügten Figuren näher erläutert. Es zeigen:
- Figur 1: eine schematische Darstellung eines stereoskopischen Endoskops;
- Figur 2: eine schematische Schnittdarstellung eines Teils eines stereoskopischen Endoskops;
- Figur 3: eine weitere schematische Schnittdarstellung des stereoskopischen Endoskops aus Figur 2;
- Figur 4: eine schematische Darstellung von Komponenten des stereoskopischen Endoskops aus den Figuren 2 und 3;
- Figur 5: eine schematische Schnittdarstellung eines weiteren optischen Instruments;
- Figur 6: ein schematisches Flussdiagramm.

### Beschreibung der Ausführungsformen

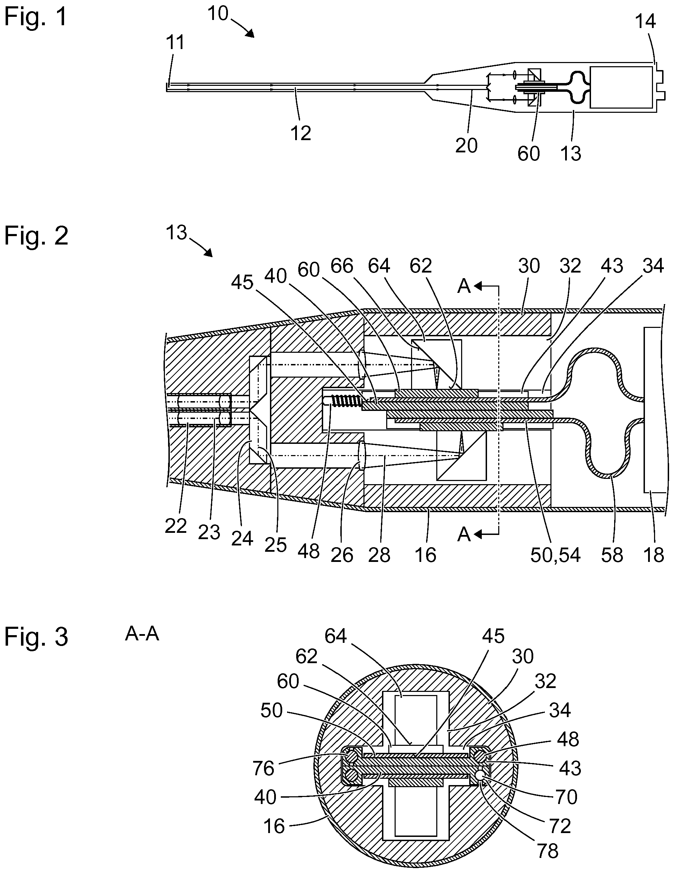

Figur 1 zeigt eine schematische Darstellung eines stereoskopischen Endoskops 10 mit einem distalen Ende 11, einem Schaft 12 und einer Handhabungseinrichtung 13 am proximalen Ende 14 des Endoskops 10. Das stereoskopische Endoskop weist zwei gleiche und symmetrisch zueinander angeordnete Beobachtungs-Strahlengänge 20 auf, von denen in Figur 1 nur einer mit einem Bezugszeichen versehen ist. Die Beobachtungs-Strahlengänge 20 sind in Figur 1 jeweils durch einfache Linien angedeutet, die insbesondere die abschnittsweise geraden optischen Achsen der den Strahlengang bildenden optischen Elemente repräsentieren. Durch kleine Pfeile ist in Figur 1 die Ausbreitungsrichtung von Licht, das von einem zu beobachtenden Gegenstand ausgeht, entlang der Beobachtungs-Strahlengänge 20 angedeutet, nämlich von distal nach proximal bzw. in Figur 1 von links nach rechts. Mittels zweier ebenfalls symmetrisch angeordneter Bildsensoren 60 (von denen in den Figuren immer nur einer mit einem Bezugszeichen versehen ist) wird das entlang der beiden Beobachtungs-Strahlengänge 20 übertragene Licht erfasst und in (insbesondere elektrische) Signale gewandelt, die zwei entsprechende Bilder repräsentieren. Bei geeigneter Wiedergabe der beiden Bilder ist eine räumliche Wahrnehmung möglich.

Figur 2 zeigt eine schematische Schnittdarstellung eines Teils einer Handhabungseinrichtung 13 eines stereoskopischen Endoskops, wie es anhand der Figur 1 dargestellt ist, oder eines Teils eines anderen optischen Instruments. Die Schnittebene der Figur 2 ist parallel zur Zeichenebene der Figur 1 und zur Längsachse des stereoskopischen Endoskops und enthält die optischen Achsen 23, 25, 28 beider Beobachtungs-Strahlengänge. Die Darstellung in Figur 2 ist gegenüber der Darstellung in Figur 1 deutlich vergrößert. Ähnlich wie in Figur 1 ist von paarweise vorgesehenen und insbesondere symmetrisch angeordneten Merkmalen in der Regel nur eines mit einem Bezugszeichen versehen.

In Figur 2 ist ein Gehäuse 16 als Beispiel einer Struktur angedeutet, die alle nachfolgend beschriebenen Elemente, Komponenten und Bestandteile unmittelbar oder mittelbar mechanisch tragen und optional gleichzeitig vor äußeren Einflüssen schützen kann. Das Gehäuse 16 kann gleichzeitig die äußere Oberfläche der Handhabungseinrichtung 13 des stereoskopischen Endoskops bilden. Alternativ kann das Gehäuse 16 für eine Anordnung in einer weiteren Hülle oder einem weiteren Gehäuse vorgesehen sein. In diesem Fall können Lichtleitfasern zur Übertragung von Beleuchtungslicht oder andere Bauteile zwischen dem Gehäuse 16 und dem weiteren Gehäuse angeordnet sein.

In der Handhabungseinrichtung 13 und bei dem dargestellten Beispiel auch innerhalb des Gehäuses 16 ist nahe dem proximalen Ende eine Steuerungs- und/oder Signalaufbereitungseinrichtung 18 angeordnet, die in Figur 2 nur teilweise sichtbar ist. Die Steuerungs- und/oder Signalaufbereitungseinrichtung 18 ist insbesondere zur Versorgung der Bildsensoren 60 mit elektrischer Leistung, zur Steuerung der Bildsensoren 60, zum Empfang von Bildsignalen von den Bildsensoren 60, zur Verstärkung und Aufbereitung (beispielsweise Digitalisierung) der Bildsignale vorgesehen und ausgebildet.

Bei dem Beispiel eines stereoskopischen Endoskops umfasst jeder Beobachtungs-Strahlengang ein Relaislinsensystem 22, das sich entlang des gesamten Schafts 12 des stereoskopischen Endoskops 10 (vgl. Figur 1) erstreckt, und dessen proximales Ende in Figur 2 sichtbar ist. Die optischen Achsen 23 der Relaislinsensysteme 22 sind parallel zueinander und zur Längsachse des stereoskopischen Endoskops 10, insbesondere seines Schafts 12, und liegen in der Schnittebene der Figur 2.

Proximal des proximalen Endes jedes Relaislinsensystems 22 ist ein Doppelprisma 24 mit zwei parallelen und gegenüber der optischen Achse 23 des Relaislinsensystems 22 insbesondere um 45 Grad geneigten reflektierenden Flächen vorgesehen. Die optischen Achsen 25 innerhalb der Doppelprismen 24 sind orthogonal zu den optischen Achsen 23 der Relaislinsensysteme 22 und liegen ebenfalls in der Schnittebene der Figur 2.

Proximal jedes Doppelprismas 24 ist ein Objektiv 26 aus einer Linse mit positiver Brechkraft oder aus mehreren Linsen mit insgesamt positiver Brechkraft als eine ein reelles Bild erzeugende optische Einrichtung vorgesehen. Die optischen Achsen 28 der Linsen oder Objektive 26 sind parallel zueinander und zu den optischen Achsen 23 der Relaislinsensysteme 22 und liegen in der Schnittebene der Figur 2. Die Doppelprismen 24 ermöglichen, dass die optischen Achsen 28 der Objektive 26 deutlich weiter voneinander beabstandet sind als die optischen Achsen 23 der Relaislinsensysteme 22. Die Linsen bzw. Objektive 26 sind insbesondere so ausgebildet und angeordnet, dass die von ihnen erzeugten reellen Bilder größer oder deutlich größer sind als die Querschnitte der Relaislinsensysteme 22 und die Zwischenbilder in den Relaislinsensystemen 22.

Die proximalen Enden der Relaislinsensysteme 22, die Doppelprismen 24 und die Linsen bzw. Objektive 26 sind in Bohrungen und/oder anderen Hohlräumen von einem oder mehreren Körpern innerhalb des Gehäuses 16 angeordnet und gehalten, gefasst oder befestigt. In einem Führungsbauteil 30 ist ein zylindrischer Hohlraum 32 vorgesehen. Der Hohlraum 32 weist beispielsweise einen teilweise rechteckigen Querschnitt auf, wobei abweichend von einem rein rechteckigen Querschnitt zwei parallele Nuten 34 parallel zur Schnittebene der Figur 2, zu den optischen Achsen 28 der Objektive 26 und zur Längsachse des optischen Instruments vorgesehen sind. Die Ränder einer der beiden Nuten, nämlich die der hinter der Schnittebene der Figur 2 liegenden Nut 34, sind in Figur 2 sichtbar. Durch die seitlichen Nuten 34 weist der Hohlraum 32 im Führungsbauteil 30 insgesamt einen im Wesentlichen kreuzförmigen Querschnitt mit unterschiedlich breiten Balken auf. Bei dem dargestellten Beispiel grenzt das Führungsbauteil distal nicht in einer Ebene an den benachbarten Körper an, sondern reicht im Bereich der Nuten 34 in den benachbarten Körper hinein.

Im Hohlraum 32 im Führungsbauteil 30 sind zwei Schlitten 40 angeordnet. Jeder Schlitten 40 ist im Wesentlichen plattenförmig und erstreckt sich primär einerseits in Richtung parallel zu den Nuten 34 und damit auch zu den optischen Achsen 28 der Linsen oder Objektive 26 und andererseits orthogonal zur Schnittebene der Figur 2. Jeder Schlitten 40 weist zwei gerade und parallele Randabschnitte 43 auf, die eine größere Dicke (gemessen in einer Richtung parallel zur Schnittebene der Figur 2 und orthogonal zu den optischen Achsen 28 der Objektive 26) aufweisen als die übrigen Bereiche des Schlittens 40. In jeder Nut 34 im Führungsbauteil 30 ist je ein gerader Randabschnitt 43 jedes Schlittens 40 angeordnet bzw. aufgenommen.

Die Schlitten 40 liegen flächig oder beispielsweise nur in den Bereichen ihrer geraden Randabschnitte 43 aneinander an. Die Randabschnitte 43 der Schlitten 40 und die Nuten 34 im Führungsbauteil 30 und insbesondere die Querschnitte (in Ebenen orthogonal zur Schnittebene der Figur 2 und zu den optischen Achsen 28 der Objektive 26) der Randabschnitte 43 und der Nuten 34 sind so ausgebildet, dass die Schlitten 40 spiel- und reibungsarm in den Nuten 34 im Führungsbauteil 30 geführt sind. Die Schlitten 40 sind somit nur in Richtung parallel zu den Nuten 34 im Führungsbauteil 30 und damit parallel zur Längsrichtung des optischen Instruments und parallel zu den optischen Achsen 28 der Objektive 26 bewegbar oder verschiebbar und weder um irgendeine Achse schwenkbar- oder rotierbar noch in einer anderen Richtung translatorisch bewegbar.

Jeder Schlitten 40 weist zwischen seinen beiden parallelen geraden Randabschnitten 43 eine Nutzfläche 45 auf. Die Nutzflächen 45 sind jeweils eben oder im Wesentlichen eben. Die Nutzflächen 45 der Schlitten 40 sind voneinander abgewandt und zueinander parallel.

Jeder Schlitten 40 weist in einem der beiden parallelen geraden Randabschnitte 43 eine durchgehende und zumindest teilweise mit einem Innengewinde versehene Bohrung parallel zum geraden Randabschnitt 43 auf, in der eine Stellschraube 48 angeordnet ist. In Figur 2 ist nur das distale Ende der Stellschraube 48 in einem der beiden Schlitten 40 sichtbar, das an einer Fläche am distalen Ende der Nut 34 im Führungsbauteil 30 anliegt. Durch Rotation der Stellschraube 48 im Uhrzeigersinn kann der zugehörige Schlitten 40 nach proximal bewegt werden. Durch eine nach distal wirkende Kraft und gleichzeitige Rotation der Stellschraube 48 gegen den Uhrzeigersinn kann der Schlitten 40 nach distal bewegt werden.

An der Nutzfläche 45 jedes Schlittens 40 ist eine Leiterplatte oder Platine 50 angeordnet. Jede Leiterplatte 50 weist elektrisch leitfähige Leiterbahnen in oder an einem elektrisch isolierenden Material auf. Jede Leiterplatte 50 weist einen an der Nutzfläche 45 eines Schlittens 40 befestigten Bereich 54 auf. Der Bereich 54 der Leiterplatte 50 kann starr oder flexibel sein und ist insbesondere durch eine vollflächige Klebeverbindung oder auf andere Weise mit dem Schlitten 40 mechanisch starr verbunden. Jede Leiterplatte 50 weist ferner einen flexiblen und gekrümmten Bereich 58 auf, dessen proximales Ende mit der Steuerungs- und/oder Signalaufbereitungseinrichtung 18 mechanisch und elektrisch verbunden ist.

An der vom Schlitten 40 abgewandten Seite jeder Leiterplatte 50 ist ein Bildsensor 60 angeordnet. Die Bildsensoren 60 sind beispielsweise CCD- oder CMOS-Sensoren. Jeder Bildsensor 60 weist eine lichtempfindliche Schicht oder Fläche 62 auf, die insbesondere durch ein rechteckiges Array oder eine matrixförmige Anordnung von Pixeln bzw. kleinen einzeln auslesbaren lichtempfindlichen Flächen gebildet wird. Im Folgenden wird beispielhaft die von der Leiterplatte 50 abgewandte Oberfläche des Bildsensors 60 als lichtempfindliche Fläche 62 bezeichnet. Vom Bildsensor 60 erzeugte elektrische Signale werden über Lötkontakte oder auf andere Weise zur Leiterplatte 50 übertragen, können durch in Figur 2 nicht dargestellte elektronische Bauelemente an oder in der Leiterplatte 50 verstärkt oder auf andere Weise verarbeitet und schließlich über den flexiblen und gekrümmten Bereich 58 der Leiterplatte 50 zur Steuerungs- und/oder Signalaufbereitungseinrichtung 18 übertragen werden.

An der lichtempfindlichen Fläche 62 jedes Bildsensors 60 ist jeweils ein Prisma 64 aus einem oder mehreren für das zu erfassende Licht transparenten Materialien angeordnet. Das Prisma 64 ist insbesondere jeweils unmittelbar auf den zugehörigen Bildsensor 60 aufgeklebt und kann so auch die lichtempfindliche Fläche 62 des Bildsensors 60 schützen. In jedem Prisma 64 ist eine reflektierende Fläche 66 vorgesehen. Die reflektierenden Flächen 66 sind jeweils gegenüber der optischen Achse 28 des Objektivs 26 und gegenüber der lichtempfindlichen Fläche 62 des Bildsensors 60 um 45 Grad geneigt. Die reflektierenden Flächen 66 werden insbesondere durch Grenzflächen zwischen Materialien mit unterschiedlichen Brechungsindizes gebildet, an denen Totalreflexion stattfindet. Alternativ können die reflektierenden Fläche 66 der Prismen 64 durch Metallschichten oder andere reflektierende Schichten oder Stapel von Schichten gebildet sein.

In Figur 2 deuten von den Rändern der Objektive 26 ausgehende durchgezogene gerade Linien die Ränder von Lichtbündeln, die von einem einzigen Objektpunkt ausgehen, an. In Figur 2 ist angedeutet, dass diese Lichtbündel in den beiden optischen Kanälen nach den Objektiven 26 unterschiedlich konvergieren. Die durch die Relaislinsensysteme 22 und die Objektive 26 erzeugten reellen Bilder befinden sich deshalb in unterschiedlichen Abständen von den Lichtaustrittsflächen der Objektive 26. Dies kann von einer Streuung der optischen Eigenschaften der verwendeten optischen Elemente und/oder von einer nicht idealen Positionierung der optischen Elemente herrühren.

Die Bewegbarkeit oder Verschiebbarkeit der Schlitten 40 zusammen mit den Bildsensoren 60 und den Prismen 64 ermöglicht eine Justage bzw. eine Kompensation nicht idealer und nicht identischer Eigenschaften beider Strahlengänge. Jeder Schlitten 40 kann einzeln durch Rotation seiner Stellschraube 48 im Uhrzeigersinn (von proximal aus gesehen) nach proximal und durch Rotation seiner Stellschraube 48 gegen den Uhrzeigersinn und gleichzeitige Erzeugung einer Kraft, die den Schlitten 40 nach distal schiebt, nach distal bewegt werden. Die Übersetzung der Rotationsbewegung durch die ineinander eingreifenden Gewinde an der Stellschraube 48 und am Schlitten 40 in eine Translationsbewegung der Stellschraube 48 und des Schlittens 40 ermöglicht eine sehr präzise Einstellung der Position jedes Schlittens 40. Die Verbindung der Bildsensoren 60 mit der Steuerungs- und/oder Signalaufbereitungseinrichtung 18 über die flexiblen und gekrümmten Bereiche 58 der Leiterplatten 50 ermöglicht dabei ohne Weiteres in jeder Position des Bildsensors 60 eine zuverlässige elektrische Verbindung zur Übertragung von elektrischer Versorgungsleistung und elektrischer Bildsignale.

Die Anordnung der Bildsensoren 60, genauer von deren lichtempfindlichen Flächen 62, parallel zu der Richtung der optischen Achsen 28 der Objektive 26 und damit parallel zu der Richtung, in der die beiden Schlitten 40 bewegbar sind, ermöglicht eine besonders kompakte Ausgestaltung der Schlitten 40. Die im Wesentlichen plattenförmige Gestalt der Schlitten 40 ist vergleichsweise einfach und präzise erzeugbar. Die ebenfalls aus dieser Anordnung der Bildsensoren 60 resultierende Anordnung der Leiterplatten 50 parallel zur Längsachse des optischen Instruments reduziert ferner den erforderlichen Bauraum im Vergleich zu einer Anordnung, bei der die Leiterplatten 50 bereits in der unmittelbaren Nähe der Bildsensoren 60 geknickt oder gekrümmt sein müssten.

Figur 3 zeigt eine weitere schematische Schnittdarstellung der anhand der Figur 2 dargestellten Handhabungseinrichtung 13. Die Schnittebene A-A der Figur 3 ist orthogonal zur Schnittebene der Figur 2, zu den optischen Achsen 28 der Objektive 26 und damit auch orthogonal zu der Richtung, in der die Schlitten 40 bewegbar sind. Die Position der Schnittebene A-A der Figur 3 ist in Figur 2 angedeutet.

In Figur 3 ist der im Wesentlichen kreuzförmige Querschnitt des Hohlraums 32 im Führungsbauteil 30 erkennbar. Ferner ist erkennbar, wie jeweils zwei gerade Randabschnitte 43 der Schlitten 40, nämlich jeweils ein gerader Randabschnitt 43 von jedem Schlitten 40, zusammen den Querschnitt einer Nut 34 so ausfüllen, dass sie gemeinsam spiel- und reibungsarm darin geführt sind. Ferner ist erkennbar, dass die Schlitten 40 jeweils im Bereich ihrer Nutzflächen 45 zwischen den geraden Randabschnitten 43 eine deutlich geringere Dicke aufweisen als an den geraden Randabschnitten 43. Bei dem dargestellten Beispiel liegen die Schlitten 40 flächig aneinander an. Alternativ können die Schlitten 40 nur in einem oder mehreren schmalen Bereichen aneinander anliegen, beispielsweise nur in zwei streifenförmigen Bereichen nahe den geraden Randabschnitten 43.

Die beschriebenen Stellschrauben 48 zum Bewegen der Schlitten 40 sind bei dem in Figur 3 oben dargestellten Schlitten 40 im rechten geraden Randabschnitt 43 und bei dem unten dargestellten Schlitten 40 im linken Randabschnitt 43 angeordnet. In den jeweils gegenüberliegenden geraden Randabschnitten sind jeweils eine Bohrung 70 und ein die Bohrung 70 längs schneidender Schlitz 72 vorgesehen. In den Bohrungen 70 sind Innengewinde vorgesehen, in die Madenschrauben 76 eingesetzt sind. Die Schnittebene A-A der Figur 3 ist jedoch distal der Madenschraube in dem in Figur 3 unten dargestellten Schlitten 40 angeordnet, so dass nur die Madenschraube 76 in der Bohrung 70 in dem in Figur 3 oben dargestellten Schlitten 40 in Figur 3 im Schnitt sichtbar ist.

Die Bohrungen 70 verjüngen sich nach distal, d.h. ihre Querschnitte nehmen nach distal ab. Durch Einschrauben der Madenschrauben 76 in die sich verjüngenden Abschnitte der Bohrungen 70 können die Schlitze 72 gegen die elastischen Kräfte des Materials der Schlitten 40 aufgeweitet und damit die Schlitten 40 im Führungsbauteil 30 durch Klemmen arretiert werden.

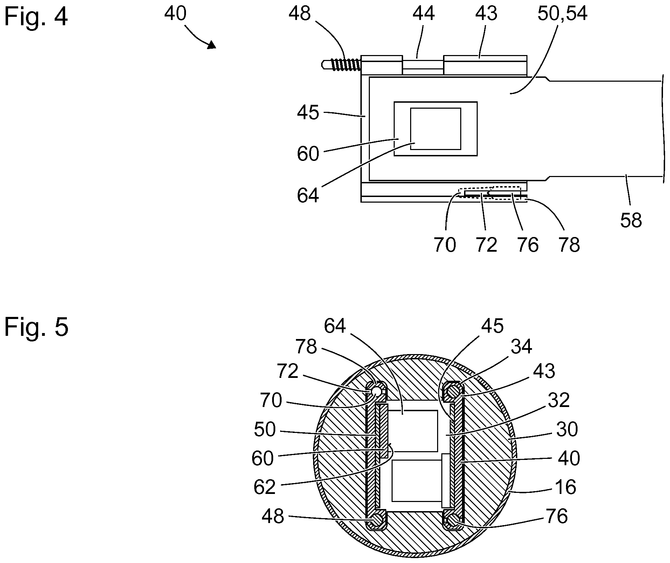

Figur 4 zeigt eine schematische Darstellung von einem der beiden Schlitten 40 aus den Figuren 2 und 3. Die Zeichenebene der Figur 4 ist orthogonal zur Schnittebene der Figur 2 und orthogonal zur Schnittebene A-A der Figur 3.

In der Draufsicht der Figur 4 ist eine Ausnehmung 44 im geraden Randabschnitt 43 des Schlittens 40 sichtbar. In der Ausnehmung 44 wird die Stellschraube 48 sichtbar. Bei dem dargestellten Beispiel weist die Stellschraube 48 nur nahe ihrem distalen Ende ein Gewinde 48 auf. Entsprechend weist die die Stellschraube 48 aufnehmende Bohrung im in Figur 4 oben dargestellten geraden Randabschnitt 43 nur distal der Ausnehmung 44 ein Innengewinde auf. An dem in Figur 4 nicht sichtbaren proximalen Ende der Stellschraube 48 ist beispielsweise ein gerader Schlitz oder ein Innen- oder Außensechskant vorgesehen, der eine formschlüssige Übertragung eines Drehmoments von einem Schraubendreher oder einem anderen geeigneten Werkzeug auf die Stellschraube 48 ermöglicht.

Am gegenüberliegenden geraden Randabschnitt 43 des Schlittens 40 sind der erwähnte Schlitz 72 und im Schlitz 72 ein Teil der Madenschraube 76 sichtbar. In gestrichelten Linien sind die nicht sichtbaren Konturen der Madenschraube 76 und der sich nach distal verjüngenden Bohrung 70 angedeutet. Eine durch den Schlitz 72 vom übrigen Schlitten 40 getrennte Zunge 78 kann durch Eindringen der Madenschraube 76 in die nach distal sich verjüngende Bohrung 70 abgespreizt und gegen die Innenwand der Nut 34 (vgl. Figuren 2, 3) gedrückt werden, um dem Schlitten 40 kraft- bzw. reibschlüssig zu arretieren.

Figur 5 zeigt eine schematische Schnittdarstellung eines weiteren optischen Instruments, das in einigen Merkmalen, Eigenschaften und Funktionen den anhand der Figuren 1 bis 3 dargestellten optischen Instrumenten ähnelt. Die Art der Darstellung, insbesondere die Schnittebene, entspricht derjenigen der Figur 3.

Das in Figur 5 gezeigte Beispiel unterscheidet sich von dem anhand der Figuren 2 und 3 dargestellten Beispiel insbesondere dadurch, dass zwei Schlitten 40 nicht flächig aneinander anliegen, sondern so voneinander beabstandet angeordnet sind, dass ihre Nutzflächen 45 sowie an den Nutzflächen 45 angeordnete Leiterplatten 50 und Bildsensoren 60 einander zugewandt sind. Die Bildsensoren 60 und darauf angeordnete Prismen 64 sind nicht wie in den Figuren 3 und 4 angedeutet mittig, sondern jeweils nahe einem Rand der zugeordneten Leiterplatte 50 angeordnet.

Der Hohlraum 32 im Führungsbauteil 30 weist keinen kreuzförmigen, sondern einen im Wesentlichen H-förmigen Querschnitt auf. Für jeden geraden Randabschnitt 43 jedes Schlittens 40 ist eine eigene Nut 34 vorgesehen. Jeder Schlitten 40 kann wie anhand der Figuren 2 bis 4 dargestellt mittels einer Stellschraube 48 entlang eines vorbestimmten Pfads orthogonal zur Zeichenebene der Figur 5 bewegt und mittels einer Madenschraube 76 durch Klemmen arretiert werden.

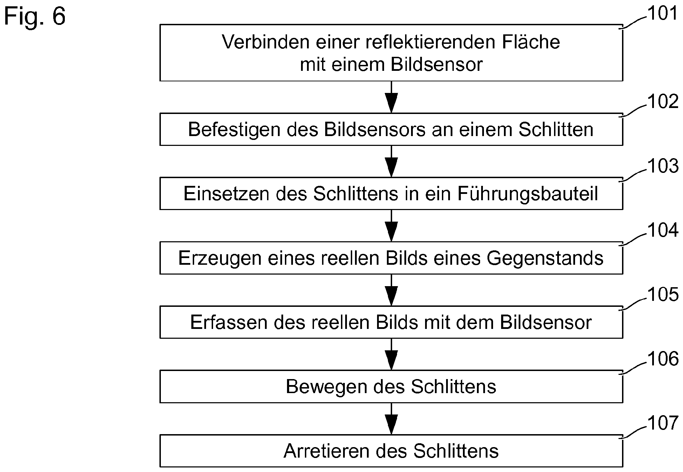

Figur 6 zeigt ein schematisches Flussdiagramm eines Teils eines Verfahrens zum Herstellen eines optischen Instruments, insbesondere eines stereoskopischen Endoskops. Das Verfahren ist auch anwendbar auf optische Instrumente mit Merkmalen und Eigenschaften, die sich von den anhand der Figuren 1 bis 5 dargestellten unterscheiden. Dennoch werden zur Vereinfachung des Verständnisses nachfolgend beispielhaft Bezugszeichen aus den Figuren 1 bis 5 verwendet.

Bei einem ersten Schritt 101 wird eine reflektierende Fläche 66 mit einem Bildsensor 60 starr verbunden. Die reflektierende Fläche 66 ist insbesondere an oder in einem Prisma 64 angeordnet, dessen Lichtaustrittsfläche beim ersten Schritt 101 insbesondere unmittelbar mittels eines transparenten Klebstoffs mit einer Lichteintrittsfläche des Bildsensors 60 verbunden wird.

Bei einem zweiten Schritt 102 wird der Bildsensor 60 unmittelbar oder mittelbar (beispielsweise über eine dazwischen angeordnete Leiterplatte 50) an einem Schlitten 40 befestigt. Der Bildsensor 60 oder eine Leiterplatte 50, an der der Bildsensor befestigt ist, wird insbesondere flächig mit einer Nutzfläche 45 des Schlittens 40 durch eine Klebung verbunden. Dabei sind insbesondere die Nutzfläche 45 des Schlittens 40 und die lichtempfindliche Fläche 62 des Bildsensors insbesondere parallel zu der Richtung, in der der Schlitten 40 bewegbar ist.

Der erste Schritt 101 und der zweite Schritt können alternativ in umgekehrter Reihenfolge ausgeführt werden.

Bei einem dritten Schritt 103 wird der Schlitten 40 in ein Führungsbauteil 30 eingesetzt. Dabei werden insbesondere zwei parallele gerade Randabschnitte 43 in zwei einander gegenüberliegende Nuten 34 im Führungsbauteil 30 eingesetzt. Beim dritten Schritt 103 können gleichzeitig oder nacheinander zwei entsprechend dem ersten Schritt 101 und dem zweiten Schritt 102 gefertigte Einheiten aus je einem Schlitten 40 und einem Bildsensor 60 in das Führungsbauteil 30 eingesetzt werden.

Bei einem vierten Schritt 104 wird mittels einer optischen Einrichtung ein reelles Bild eines Gegenstands, der sich an einer vorbestimmten Position befindet, erzeugt. Die optische Einrichtung umfasst insbesondere ein Objektiv 26 und optional ein Relaislinsensystem 22. Bei einem fünften Schritt 105 wird das beim vierten Schritt 104 erzeugte reelle Bild mittels eines Bildsensors 60 erfasst. Bei einem sechsten Schritt 106 wird der Schlitten 40 bewegt oder verschoben, bis der Bildsensor 60 das reelle Bild scharf erfasst.

Der vierte Schritt 104, der fünfte Schritt 105 und der sechste Schritt 106 können gleichzeitig ausgeführt werden. Der vierte Schritt 104, der fünfte Schritt 105 und der sechste Schritt 106 bilden ein Verfahren zum Justieren des Bildsensors 60, bei dem Fehler der das reelle Bild erzeugenden optischen Einrichtungen 22, 26 korrigiert werden können.

Bei einem siebten Schritt 107 wird der Schlitten 40 bei der Position, bei der der Bildsensor 60 das reelle Bild scharf erfasst, arretiert, insbesondere durch Klemmen.

### Bezugszeichen

- 10: stereoskopisches Endoskop als optisches Instrument
- 11: distales Ende des Endoskops 10
- 12: Schaft des Endoskops 10
- 13: Handhabungseinrichtung des Endoskops 10
- 14: proximales Ende des Endoskops 10
- 16: (äußeres oder inneres) Gehäuse der Handhabungseinrichtung 13
- 18: Steuerungs- und/oder Signalaufbereitungseinrichtung des stereoskopischen Endoskops 10
- 20: Beobachtungs-Strahlengang im Endoskop 10
- 22: Relaislinsensystem des Instruments 10
- 23: optische Achse des Relaislinsensystems 22
- 24: Doppelprisma zum seitlichen Versetzen der optischen Achse
- 25: optische Achse im Doppelprisma 24
- 26: Objektiv als ein reelles Bild erzeugende optische Einrichtung
- 28: optische Achse der optischen Einrichtung 26 nach deren Lichtaustrittsfläche 27
- 30: Führungsbauteil
- 32: Hohlraum im Führungsbauteil 30
- 34: Nut als Teil des Hohlraums im Führungsbauteil 30
- 40: Schlitten
- 43: gerader Randabschnitt des Schlittens 40, in die Nut 34 eingreifend
- 44: Ausnehmung im geraden Randabschnitt 43 des Schlittens 40
- 45: Nutzfläche des Schlittens 40
- 48: Stellschraube am Schlitten 40
- 50: Leiterplatte bzw. Platine
- 54: an der Nutzfläche 45 des Schlittens 40 befestigter Bereich der Leiterplatte 50
- 58: flexibler und gekrümmter Bereich der Leiterplatte 50
- 60: Bildsensor zum Erzeugen eines Bildsignals
- 62: lichtempfindliche Fläche des Bildsensors 60
- 64: Prisma an der lichtempfindlichen Fläche 62
- 66: reflektierende Fläche im Prisma 64
- 70: Bohrung
- 72: Schlitz im Schlitten 40 parallel zum Längsrand 43 des Schlittens 40
- 76: Madenschraube im Gewinde 74 im Schlitten 40
- 78: Zunge
- 101: erster Schritt (Befestigen eines Bildsensors an einem Schlitten)
- 102: zweiter Schritt (starres Verbinden einer reflektierenden Fläche mit dem Bildsensor)
- 103: dritter Schritt (Einsetzen des Schlittens in ein Führungsbauteil)
- 104: vierter Schritt (Erzeugen eines reellen Bilds eines Gegenstands)
- 105: fünfter Schritt (Erfassen des reellen Bilds mittels des Bildsensors)
- 106: sechster Schritt (Bewegen des Schlittens bis das reelle Bild scharf erfasst wird)
- 107: siebter Schritt (Arretieren des Schlittens)

## Patentansprüche

1. Optisches Instrument (10) zur Erfassung von Stereobildern, mit:
zwei optischen Einrichtungen (26) zum Erzeugen je eines reellen Bilds;
zwei am oder nahe dem proximalen Ende (14) des optischen Instruments (10) angeordneten Schlitten (40), die jeweils relativ zu den optischen Einrichtungen (26) entlang eines vorbestimmten Pfads bewegbar und arretierbar sind;
wobei jeder der Schlitten (40) nur in Richtung parallel zur Längsachse des optischen Instruments (10) bewegbar ist,
zwei Bildsensoren (60) mit je einer lichtempfindlichen Fläche (62) zum Erfassen des jeweiligen reellen Bilds, wobei an den Schlitten (40) je einer der Bildsensoren (60) befestigt ist;
jeweils einer reflektierenden Fläche (66) im Strahlengang zwischen der optischen Einrichtung (26) und dem Bildsensor (60).

2. Optisches Instrument (10) nach dem vorangehenden Anspruch, bei dem die reflektierenden Flächen (66) mit dem jeweiligen Bildsensor (60) starr verbunden und zusammen mit dem Bildsensor (60) bewegbar sind.

3. Optisches Instrument (10) nach einem der vorangehenden Ansprüche, bei dem die Schlitten (40) in einer Richtung parallel zu der jeweiligen lichtempfindlichen Fläche (62) des Bildsensors (60) bewegbar sind.

4. Optisches Instrument (10) nach einem der vorangehenden Ansprüche, bei dem jeweils die lichtempfindliche Fläche (62) des Bildsensors (60) parallel zur optischen Achse (28) der optischen Einrichtung (26) angeordnet ist.

5. Optisches Instrument (10) nach einem der vorangehenden Ansprüche, ferner mit:
zwei Klemmeinrichtungen (72, 74, 76) zum kraftschlüssigen Arretieren je eines der Schlitten (40).

6. Optisches Instrument (10) nach einem der vorangehenden Ansprüche, ferner mit:
wenigstens einer Stellschraube (48) zum Bewegen eines der Schlitten (40).

7. Optisches Instrument (10) nach einem der vorangehenden Ansprüche, ferner mit:
wenigstens einer Leiterplatte (50), die mit einem der Schlitten (40) verbunden ist, wobei der jeweilige Bildsensor (60) an der Leiterplatte (50) befestigt ist,
wobei die Leiterplatte (50) einen flexiblen gekrümmten Abschnitt (58) aufweist, der einen Längenausgleich bei Bewegung des Schlittens (40) ermöglicht.

8. Optisches Instrument (10) nach einem der vorangehenden Ansprüche, bei dem das optische Instrument (10) ein Endoskop mit einem starren Schaft (12) ist.

9. Optisches Instrument (10) nach dem vorangehenden Anspruch, bei dem die optische Einrichtung (26) mindestens ein Stablinsensystem oder ein anderes Relaislinsensystem umfasst.

10. Optisches Instrument (10) nach einem der vorangehenden Ansprüche, ferner mit:
einem Führungsbauteil (30), das mit den optischen Einrichtungen (26) starr verbunden ist,
wobei das Führungsbauteil (30) zwei Nuten (34) aufweist, in denen zwei von einander abgewandte und parallele gerade Randabschnitte (43) zumindest eines der Schlitten (40) geführt sind.

11. Optisches Instrument (10) nach Anspruch 10, bei dem in jeder der zwei Nuten (34) je ein Randabschnitt (43) von jedem der zwei Schlitten (40) nebeneinander angeordnet ist.

12. Optisches Instrument (10) nach einem der vorangehenden Ansprüche, bei dem die zwei Bildsensoren (60) an voneinander abgewandten Seiten der zwei Schlitten (40) angeordnet sind.

13. Verfahren zum Justieren eines Bildsensors (60) eines optischen Instruments (10), mit folgendem Schritt:
Erzeugen (104) eines Bilds eines an einer vorbestimmten Position angeordneten Gegenstands;
Bewegen (106) des Bildsensors (60) bis zu einer Position, bei der der Bildsensor (60) das Bild scharf erfasst;
Arretieren (107) eines Schlittens (40) mit dem Bildsensor (60) bei der Position, bei der der Bildsensor (60) das Bild scharf erfasst,
wobei bei dem Schritt (106) des Bewegens der Bildsensor (60) mit dem Schlitten (40) parallel zu einer lichtempfindlichen Oberfläche (62) des Bildsensors (60) und parallel zu einer Längsachse des Instruments (10) bewegt wird.

## Claims

1. Optical instrument (10) for capturing stereo images, comprising:
two optical devices (26) for producing a real image in each case;
two carriages (40) arranged at or near the proximal end (14) of the optical instrument (10), said carriages each being movable along a predetermined path relative to the optical devices (26) and lockable;
each of the carriages (40) being movable only in the direction parallel to the longitudinal axis of the optical instrument (10),
two image sensors (60), each with a light-sensitive surface (62) for capturing the respective real image, with one of the image sensors (60) in each case being fastened to the carriages (40);
one reflecting surface (66) in the beam path between the optical device (26) and the image sensor (60) in each case.

2. Optical instrument (10) according to the preceding claim, wherein the reflecting surfaces (66) are rigidly connected to the respective image sensor (60) and movable together with the image sensor (60).

3. Optical instrument (10) according to one of the preceding claims, wherein the carriages (40) are movable in a direction parallel to the respective light-sensitive surface (62) of the image sensor (60).

4. Optical instrument (10) according to one of the preceding claims, wherein the light-sensitive surface (62) of the image sensor (60) is arranged parallel to the optical axis (28) of the optical device (26) in each case.

5. Optical instrument (10) according to one of the preceding claims, further comprising:
two clamping devices (72, 74, 76) for locking respectively one of the carriages (40) in a force-fit manner.

6. Optical instrument (10) according to one of the preceding claims, further comprising:
at least one setscrew (48) for moving one of the carriages (40).

7. Optical instrument (10) according to one of the preceding claims, further comprising:
at least one printed circuit board (50) connected to one of the carriages (40), with the respective image sensor (60) being fastened to the printed circuit board (50),
the printed circuit board (50) having a flexible curved portion (58) which facilitates a length compensation when moving the carriage (40).

8. Optical instrument (10) according to one of the preceding claims, wherein the optical instrument (10) is an endoscope with a rigid shank (12).

9. Optical instrument (10) according to the preceding claim, wherein the optical device (26) comprises at least one rod lens system or another relay lens system.

10. Optical instrument (10) according to one of the preceding claims, further comprising:
a guide component (30) which is rigidly connected to the optical devices (26),
the guide component (30) having two grooves (34), in which two parallel straight edge portions (43), which face away from one another, of at least one of the carriages (40) are guided.

11. Optical instrument (10) according to Claim 10, wherein respectively one edge portion (43) from each one of the two carriages (40) is arranged, one next to the other, in each one of the two grooves (34).

12. Optical instrument (10) according to one of the preceding claims, wherein the two image sensors (60) are arranged on sides facing away from one another of the two carriages (40).

13. Method for adjusting an image sensor (60) of an optical instrument (10), comprising the following step:
producing (104) an image of an object arranged at a predetermined position;
moving (106) the image sensor (60) to a position in which the image sensor (60) captures the image in focus;
locking (107) a carriage (40) with the image sensor (60) at the position in which the image sensor (60) captures the image in focus,
wherein the image sensor (60) is moved with the carriage (40) parallel to a light-sensitive surface (62) of the image sensor (60) and parallel to a longitudinal axis of the instrument (10) within the step of moving (106).

## Revendications

1. Instrument optique (10) destiné à l'acquisition d'images stéréoscopiques, comportant :
deux dispositifs optiques (26) chacun destinés à générer une image réelle ;
deux chariots (40) disposés à l'extrémité proximale (14) de l'instrument optique (10) ou à proximité de celle-ci, chaque chariot pouvant être déplacé et bloqué le long d'un trajet prédéterminé par rapport aux dispositifs optiques (26) ;
dans lequel chacun des chariots (40) n'est mobile que dans une direction parallèle à l'axe longitudinal de l'instrument optique (10),
deux capteurs d'image (60) présentant chacun une surface photosensible (62) pour l'acquisition de l'image réelle respective, dans lequel les capteurs d'image (60) sont chacun fixés aux chariots (40) ;
une surface réfléchissante (66) respective sur le trajet du faisceau entre le dispositif optique (26) et le capteur d'image (60).

2. Instrument optique (10) selon la revendication précédente, dans lequel les surfaces réfléchissantes (66) sont reliées rigidement au capteur d'image (60) respectif et peuvent être déplacées avec le capteur d'image (60).

3. Instrument optique (10) selon l'une des revendications précédentes, dans lequel les chariots (40) peuvent être déplacés dans une direction parallèle à la surface photosensible (62) respective du capteur d'image (60).

4. Instrument optique (10) selon l'une des revendications précédentes, dans lequel la surface photosensible (62) du capteur d'image (60) respective est agencée parallèlement à l'axe optique (28) du dispositif optique (26).

5. Instrument optique (10) selon l'une des revendications précédentes, comportant en outre :
deux dispositifs de serrage (72, 74, 76) chacun destinés à bloquer l'un des chariots (40) par complémentarité de force.

6. Instrument optique (10) selon l'une des revendications précédentes, comportant en outre :
au moins une vis de réglage (48) destinée à déplacer l'un des chariots (40).

7. Instrument optique (10) selon l'une des revendications précédentes, comportant en outre :
au moins une carte de circuit imprimé (50) reliée à l'un des chariots (40), dans lequel le capteur d'image (60) respectif est fixé à la carte de circuit imprimé (50),
dans lequel la carte de circuit imprimé (50) présente une partie incurvée flexible (58) qui permet une compensation de longueur lors du déplacement du chariot (40) .

8. Instrument optique (10) selon l'une des revendications précédentes, dans lequel l'instrument optique (10) est un endoscope comportant un tube rigide (12) .

9. Instrument optique (10) selon la revendication précédente, dans lequel le dispositif optique (26) comprend au moins un système de lentille barreau ou un autre système de lentille relais.

10. Instrument optique (10) selon l'une des revendications précédentes, comportant en outre :
un élément de guidage (30) relié rigidement au dispositif optique (26),
dans lequel l'élément de guidage (30) présente deux rainures (34) dans lesquelles sont guidées deux parties de bord droites (43), tournées à l'opposé l'une de l'autre, d'au moins l'un des chariots (40).

11. Instrument optique (10) selon la revendication 10, dans lequel des parties de bord (43) de chacun des deux chariots (40) sont agencées côte à côte dans chacune des deux rainures (34).

12. Instrument optique (10) selon l'une des revendications précédentes, dans lequel les deux capteurs d'image (60) sont agencés sur des côtés tournés à l'opposé l'un de l'autre des deux chariots (40) .

13. Procédé pour l'ajustement d'un capteur d'image (60) d'un instrument optique (10), comprenant les étapes consistant à :
générer (104) une image d'un objet agencé à une position prédéterminée ;
déplacer (106) le capteur d'image (60) jusqu'à une position à laquelle le capteur d'image (60) détecte l'image de manière nette ;
bloquer (107) un chariot (40) comportant le capteur d'image (60) à la position à laquelle le capteur d'image (60) détecte l'image de manière nette,
dans lequel, lors de l'étape de déplacement (106), le capteur d'image (60) est déplacé avec le chariot (40) parallèlement à une surface photosensible (62) du capteur d'image (60) et parallèlement à un axe longitudinal de l'instrument (10).
